Europäisches Patentamt
European Patent Office
Office européen des brevets

(11) Veröffentlichungsnummer: **0 355 384 A1**

# EUROPÄISCHE PATENTANMELDUNG

(21) Anmeldenummer: **89113007.2**

(22) Anmeldetag: **15.07.89**

(51) Int. Cl.⁴: **C07C 309/42 , C07C 303/00**

(30) Priorität: **22.07.88 DE 3824901**

(43) Veröffentlichungstag der Anmeldung: **28.02.90 Patentblatt 90/09**

(84) Benannte Vertragsstaaten: **DE FR GB**

(71) Anmelder: **HOECHST AKTIENGESELLSCHAFT**
**Postfach 80 03 20**
**D-6230 Frankfurt am Main 80(DE)**

(72) Erfinder: **Gethöffer, Hanspeter, Dr.**
**Geisenheimer Strasse 97**
**D-6000 Frankfurt am Main 71(DE)**
Erfinder: **Grabley, Fritz-Feo, Dr.**
**Hölderlinstrasse 7**
**D-6240 Königstein/Taunus(DE)**

## (54) Verfahren zur Herstellung von Acyloxibenzolsulfonsäuren oder deren Salzen.

(57) Verfahren zur Herstellung von Acyloxibenzolsulfonsäuren oder deren Salzen der Formel

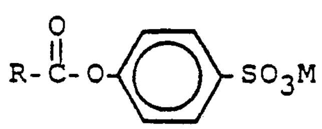

durch gleichzeitige Umsetzung von Hydroxibenzolsulfonsäure oder deren Salzen mit Acetanhydrid und einer Carbonsäure der Formel
R-COOH.

Nach Beendigung der Reaktion wird die Acyloxibenzolsulfonsäure in reiner Form erhalten durch Auswaschen des Reaktionsgemisches mit einem hydrophilen Lösemittel, vorzugweise mit einem $C_1$-$C_3$-Alkohol.

EP 0 355 384 A1

## Verfahren zur Herstellung von Acyloxibenzolsulfonsäuren oder deren Salzen

Die Erfindung betrifft ein Verfahren zur Herstellung von Acyloxibenzolsulfonaten ausgehend von Phenolsulfonaten, Carbonsäuren und Acetanhydrid.

Acyloxibenzolsulfonate sind seit langem bekannte Verbindungen mit Perborat-aktivierenden Eigenschaften, für die es mehrere Möglichkeiten zur Herstellung gibt.

In DRP 666 626 ist ein Verfahren beschrieben, bei dem Ester aus Phenolen und Carbonsäuren mit einem Sulfierungsmittel umgesetzt und die Sulfonsäuren anschließend neutralisiert werden. Diese Estersulfierung wird mit speziellen Verfahrensschritten in weiteren Patentanmeldungen beschrieben, z.B. in EP 163 224, EP 165 480, EP 201 222 und EP 227 194.

Aus US-A 3 503 888 ist ein Verfahren bekannt, bei dem Phenol sulfiert, dann mit einem Fettsäurechlorid verestert und anschließend neutralisiert wird. Auf dieser Basis werden spezielle Varianten dieses Verfahrens in weiteren Patentanmeldungen beschrieben, z.B. in EP 163 225, EP 207 445, EP 220 656 und EP 229 890.

Diese beiden grundsätzlichen Methoden haben jedoch einen entscheidenden Nachteil, wenn es um die Herstellung besonders hochprozentiger und definierter 4-Acyloxibenzolsulfonate geht. Verfahrensbedingt entstehen neben dem gewünschten 4-Isomeren auch mehr oder weniger große Mengen des 2-Isomeren sowie andere Nebenprodukte.

Dieses Problem kann grundsätzlich dadurch gelöst werden, daß bei der Herstellung der Acyloxibenzolsulfonate von definierten 4-Hydroxybenzolsulfonaten und Carbonsäurechlorid ausgegangen wird, wie dies schon aus DRP 657 357 bekannt ist. Dieser Weg wird in einer Reihe von Patentanmeldungen beschrieben, z.B. in EP 98 129, EP 148 148, EP 164 786 und EP 220 826. Außerdem gibt es eine Reihe von Verfahren, die statt des Carbonsäurechlorids das Carbonsäureanhydrid oder den Carbonsäurephenylester als Acylierungsmittel einsetzen, wie z.B. in EP 105 672, EP 105 673, EP 125 641, EP 153 222 und EP 153 223 beschrieben.

Problematisch bei dieser Verfahrensweise ist jedoch, daß diese Reaktionen meist Gleichgewichtsreaktionen sind und besondere Maßnahmen nötig sind, um hohe Ausbeuten und Reinheiten zu erzielen.

Die EP 105 672 beschreibt ein Eintopfverfahren, bei dem 4-Hydroxibenzolsulfonat gleichzeitig mit Acetanhydrid und der Carbonsäure umgesetzt wird. Die Abtrennung und Rückführung der überschüssigen Carbonsäure erfolgt dabei durch Waschen mit einem hydrophoben Lösungsmittel wie Ether oder Hexan. Ein Nachteil dieses Verfahrens ist, daß das Zwischenprodukt 4-Acetoxibenzolsulfonat in diesen Lösungsmitteln unlöslich ist und daher bei unvollständiger Umsetzung im Produkt verbleibt. Ein nach diesem Verfahren hergestelltes 4-Acyloxibenzolsulfonat erfüllt aber nicht die Anforderungen, die an ein hochprozentiges und sehr reines Produkt gestellt werden. Ein weiterer Nachteil dieses Verfahrens ist, daß das eingesetzte 4-Hydroxibenzolsulfonat aufwendig und kostenintensiv feinstgemahlen werden muß (Teilchendurchmesser <100 µ), um eine vollständige Umsetzung zu 4-Acyloxibenzolsulfonaten zu erreichen. Andernfalls verbleibt auch dann ein Teil des Zwischenproduktes 4-Acetoxibenzolsulfonat im Endprodukt.

Aufgabe der vorliegenden Erfindung ist es deshalb, ein verbessertes Verfahren aufzuzeigen, das in guten Ausbeuten zu äußerst reinem 4-Acyloxibenzolsulfonat führt.

Gegenstand der Erfindung ist somit ein Verfahren zur Herstellung von Acyloxibenzolsulfonsäuren oder deren Salzen der Formel

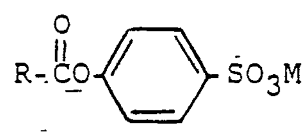

worin R $C_5$-$C_{21}$-Alkyl, $C_5$-$C_{21}$-Alkenyl, α-mono-Chlor-$C_5$-$C_{21}$-Alkyl, ω-$C_1$-$C_4$-Alkoxi-$C_5$-$C_{21}$-alkyl oder Phenyl das durch 1 bis 3 Substituenten aus der Gruppe F, Cl, $SO_3M$, COOM, $C_1$-$C_{21}$-Alkyl und $C_2$-$C_{20}$-Alkenyl substituiert sein kann, und M Wasserstoff, Ammonium, ein Alkalimetallatom oder das Äquivalent eines Erdalkalimetallatoms bedeuten, durch Umsetzung von Hydroxibenzolsulfonsäure oder deren Salzen der Formel

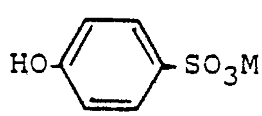

mit Acetanhydrid und einer Carbonsäure der Formel

R-COOH

wobei R und M die oben genannten Bedeutungen haben, unter gleichzeitigem Abdestillieren der entstehenden Essigsäure und anschließender Isolierung der Acyloxibenzolsulfonsäure oder deren Salze, wobei man die Acyloxibenzolsulfonsäure in der Weise aus dem Reaktionsgemisch isoliert, daß man nach Beendigung der Reaktion den Rückstand mit einem hydrophilen Lösemittel auswäscht.

Bevorzugt werden Acyloxibenzolsulfonsäuren

oder deren Salze der obigen Formel hergestellt, worin R $C_5$-$C_{11}$-Alkyl, $C_5$-$C_{11}$-Alkenyl, α-mono-Chlor-$C_5$-$C_{11}$-Alkyl, ω-$C_1$-C4-Alkoxy-$C_5$-$C_{11}$-alkyl oder Phenyl bedeuten.

Zur Durchführung der Reaktion wird ein Gemisch aus Hydroxibenzolsulfonat, Acetanhydrid und einer Carbonsäure der angegebenen Formel im molaren Verhältnis 1 : 1-2 : 1-5, bevorzugt 1 : 1,2-1,5 : 2-4 umgesetzt. Zur Beschleunigung der Reaktion kann als Katalysator ein Alkali- oder Erdalkalisalz einer Carbonsäure, beispielsweise Natriumacetat, zugegeben werden. Es ist auch möglich, zunächst in einem separaten Schritt das Acetoxibenzolsulfonat herzustellen und dieses Produkt dann mit 1 bis 5, vorzugsweise 2 bis 4 Mol der angegebenen Carbonsäuren umzuestern.

Carbonsäuren, die in dem erfindungsgemäßen Verfahren eingesetzt werden können, sind u.a. Hexansäure, Octansäure, Nonansäure, Laurinsäure, Benzoesäure, Isononansäure, Chlorbenzoesäure. Die Reaktionstemperatur muß so hoch sein, daß die im Verlaufe der Reaktion gebildete Essigsäure abdestillieren kann. Dies ist im allgemeinen bei Temperaturen von 120-250 °C, bevorzugt bei 150-200 °C der Fall. Die Reaktionsdauer richtet sich nach der Art der Carbonsäure und des Katalysators.

Nach Beendigung der Reaktion läßt man das Reaktionsgemisch abkühlen und entfernt die überschüssige Carbonsäure sowie Restmengen an Acetoxibenzolsulfonat, die nicht umgeestert worden sind, durch Waschen mit einem hydrophilen Lösemittel, bevorzugt mit einem $C_1$-$C_3$-Alkohol. Die dabei anfallende alkoholische Phase enthält somit die Carbonsäure und Acetoxibenzolsulfonat neben gewissen Anteilen an Acyloxibenzolsulfonat. Es ist deshalb sinnvoll, das hydrophile Lösemittel abzudestillieren und den Rückstand erneut bei der Reaktion, wie oben beschrieben, einzusetzen. Die gesamte Reaktion kann somit auch nach bekannten Methoden kontinuierlich ausgestaltet sein.

Das erfindungsgemäße Verfahren weist folgende Vorteile gegenüber dem Stand der Technik auf:
- Hohe Reinheitsgrade der erhaltenen Produkte auch bei unvollständigem Umsatz.
- Durch Wiedereinsatz des gesamten Rückstandes werden hohe Ausbeuten erzielt.
- Essigsäure als Nebenprodukt kann erneut in Acetanhydrid umgewandelt werden.

**Beispiele**

Vergleichsbeispiel (nach EP-A 0 105 672):

Natriumnonanoyloxibenzolsulfonat

In einer Apparatur, bestehend aus 1 l-Vierhalskolben mit Innenthermometer und 30 cm Vigreuxkolonne mit Destillationsbrücke werden unter Stickstoff (Stickstoffzuleitung über Kopf der Vigreuxkolonne) 79,1 g (0,5 Mol) Nonansäure, 19,6 g (0,1 Mol) wasserfreies Natriumphenolsulfonat, 12,25 g (0,12 Mol) Essigsäureanhydrid und 1 g Natriumacetat während 40 min auf 165 °C erhitzt und 140 min bei 165-170 °C unter Rückfluß gehalten. Dann wird die Stickstoffzufuhr am Kolonnenkopf unterbrochen und zur Unterstützung der Destillation durch den Kolben geleitet. Unter ständigem Abdestillieren von Essigsäure steigt die Temperatur während 70 min auf 220 °C. Der Rückstand wird zweimal mit je 1 l Diethylether gewaschen und im Vakuum bei 80 °C getrocknet.
Ausbeute: 30,75 g (91,4 %)
Wirksubstanz (WS)-Gehalt: 84 % (Mischindikatortitration)

**Beispiel 1**

Natriumnonanoyloxibenzolsulfonat

1. Ansatz

In einer Apparatur, bestehend aus 1 l-Vierhalskolben mit Innenthermometer umd 30 cm Vigreuxkolonne mit Destillationsbrücke werden unter Stickstoff (Stickstoffzuleitung über Kopf der Vigreuxkolonne) 158 g (1 Mol) Nonansäure, 61,25 g (0,6 Mol) Acetanhydrid, 98,1 g (0,5 Mol) Natriumphenolsulfonat und 2 g Natriumacetat während 40 min auf 170-180 °C erhitzt. Dabei destilliert ab 165 °C Innentemperatur Essigsäure über. Dieser Temperaturbereich wird 3 h gehalten, dann die Stickstoffzufuhr am Kolonnenkopf unterbrochen und Stickstoff zur Unterstützung der Destillation durch den Kolben geleitet. Unter ständigem Abdestillieren von Essigsäure steigt die Temperatur im Kolben auf 225 °C. Es wird noch 10 min gerührt und dann auf Raumtemperatur abgekühlt. Der Rückstand wird mit 500 ml Methanol versetzt, 20 min gerührt und Methanol abfiltriert. Nach 2-maligem Waschen des Rückstandes mit je 200 ml Methanol wird im Vakuum bei 80 °C getrocknet.
Ausbeute: 108,5 g (65 %)
WS-Gehalt:97 % (Mischindikatortitration)

Die vereinigten Methanolfiltrate werden soweit wie möglich eingeengt; es werden 157 g Rückstand folgender Zusammensetzung (Bestimmung der Acyloxibenzolsulfonate durch quantitative HPLC) erhalten:
28 g (0,08 Mol) Natriumnonanoyloxibenzolsulfonat
25 g (0,1 Mol) Natriumacetoxibenzolsulfonat

95 g (0,6 Mol) Nonansäure
2 g Natriumacetat
7 g Methanol

2. Ansatz

98,10 g (0,5 Mol) Natriumphenolsulfonat
79,10 g (0,5 Mol) Nonansäure
61,25 g (0,6 Mol) Acetanhydrid
157,00 g Rückstand aus Ansatz 1

Alle angegebenen Komponenten außer Acetanhydrid werden zusammengegeben. Beim Erhitzen auf 130 °C destilliert restliches Methanol ab. Acetanhydrid wird dann zugegeben und während 40 min auf 170 °C erhitzt. Die weitere Reaktionsführung und Aufarbeitung erfolgt wie in Ansatz 1 angegeben.

Ausbeute: 147 g
WS-Gehalt: 97 %
Natriumnonanoyloxibenzolsulfonat
Gs. Ausbeute nach 2 Ansätzen 255,5 g (≙ 76 % bezogen auf 1 Mol Natriumphenolsulfonat)
Rückstand aus Methanol: 176,0 g
bestehend aus:
40,3 g (0,12 Mol Natriumnonanoyloxibenzolsulfonat
30,0 g (0,12 Mol) Natriumacetoxibenzolsulfonat
100,0 g (0,63 Mol) Nonansäure
3,7 g Methanol
2,0 g Natriumacetat

3. Ansatz

98,10 g (0,5 Mol) Natriumphenolsulfonat
79,10 g (0,5 Mol) Nonansäure
61,25 g (0,6 Mol) Acetanhydrid
176,00 g Rückstand aus Methanol (aus Ansatz 2)
Ausbeute: 151 g
WS-Gehalt: 96 %
Ges. Ausbeute nach 3 Ansätzen: 406,5 g Natriumnonanoyloxibenzolsulfonat (≙ 80,1 % bezogen auf 1,5 Mol Natriumphenolsulfonat)
Rückstand aus Methanol: 176,3 g
bestehend aus:
50,4 g (0,15 Mol) Natriumnonanoyloxibenzolsulfonat
35,1 g (0,15 Mol) Natriumacetoxibenzolsulfonat
85,3 g (0,54 Mol) Nonansäure
3,5 g Methanol
2,0 g Natriumacetat

**Beispiel 2**

Natriumlauroyloxibenzolsulfonat

1. Ansatz

Unter Stickstoff werden 120,2 g (0,60 Mol) Laurinsäure, 58,85 g (0,30 Mol) Natriumphenolsulfonat, 36,75 g (0,36 Mol) Acetanhydrid und 2 g Natriumacetat während 40 min auf 155 °C und während 75 min auf 175-180 °C erhitzt. Dabei destilliert ab 165 °C Essigsäure über. Dieser Temperaturbereich wird über 4 Stunden gehalten, dann die Stickstoffzufuhr am Kolonnenkopf unterbrochen und Stickstoff zur Unterstützung der Destillation durch den Kolben geleitet. Unter ständigem Abdestillieren von Essigsäure steigt die Temperatur im Kolben auf 230 °C. Nach Abkühlen wird der Rückstand mit 300 ml Methanol versetzt, 20 min gerührt und das Methanol abfiltriert. Nach zweimaligem Waschen des Rückstandes mit je 150 ml Methanol wird im Vakuum bei 80 °C getrocknet.

Aubeute: 83 g (73 %)
WS-Gehalt: 93 %

Die vereinigten Methanolfiltrate werden soweit wie möglich eingeengt. Es werden 109,7 g Rückstand erhalten, bestehend aus:

9,8 g (0,04 Mol) Natriumacetoxibenzolsulfonat
73,3 g (0,37 Mol) Laurinsäure
15,1 g (0,04 Mol) Natriumlauroyloxibenzolsulfonat
2,0 g Natriumacetat
5,0 g Methanol

2. Ansatz

60,05 g (0,3 Mol) Laurinsäure
58,85 g (0,3 Mol) Natriumphenolsulfonat
36,75 g (0,36 Mol) Acetanhydrid
105,2 g Rückstand aus Ansatz 1

Es werden alle angegebenen Komponenten außer Acetanhydrid zusammengegeben. Beim Erhitzen auf 130 °C destilliert restliches Methanol ab. Acetanhydrid wird dann zugegeben und während 40 min auf 170 °C erhitzt. Zur weiteren Reaktionsführung und Aufarbeitung siehe Ansatz 1.

Ausbeute: 115 g
WS-Gehalt: 92 %
Ges. Ausbeute nach 2 Ansätzen: 198 g Natriumlauroyloxibenzolsulfonat (87 % bezogen auf 0,6 Mol Natriumphenolsulfonat)
Rückstand aus Methanol: 116,4 g
bestehend aus:
11,6 g (0,05 Mol) Natriumacetoxibenzolsulfonat
76,1 g (0,38 Mol) Laurinsäure
10,5 g (0,03 Mol) Natriumlauroyloxibenzolsulfonat
2,0 g Natriumacetat
7,0 g Methanol

3. Ansatz

60,05 g (0,3 Mol) Laurinsäure
58,85 g (0,3 Mol) Natriumphenolsulfonat
36,75 g (0,36 Mol) Acetanhydrid
116,4 g Rückstand aus Methanol (aus Ansatz 2)
Ausbeute: 118 g
WS-Gehalt: 94 %
Ges. Ausbeute nach 3 Ansätzen: 316 g Natriumlauroyloxibenzolsulfonat (92,7 % bezogen auf 0,9 Mol Natriumphenolsulfonat)
Rückstand aus Methanol: 108,6 g
bestehend aus
12,8 g (0,054 Mol) Natriumacetoxibenzolsulfonat
70,1 g (0,35 Mol) Laurinsäure
19,7 g (0,049 Mol) Natriumlauroyloxibenzolsulfonat
2,0 g Natriumacetat
4,0 g Methanol

**Beispiel 3**

Natriumisononanoyloxibenzolsulfonat

1. Ansatz

Unter Stickstoff werden 127,8 g (0,81 Mol) Isononansäure, 36,75 g (0,36 Mol) Acetanhydrid, 58,85 g (0,3 Mol) Natriumphenolsulfonat und 2 g Natriumacetat während 30-35 Minuten auf 160-165 °C erhitzt und 3 h 45 min bei dieser Temperatur gehalten. Es wird während 1 h auf 260 °C erhitzt. Dabei destilliert Essigsäure nahezu vollständig über. Es wird noch 45 min bei dieser Temperatur gehalten und nach Abkühlen auf Raumtemperatur der Rückstand mit 500 ml Ethanol versetzt, 20 min gerührt und abfiltriert. Nach zweimaligem Waschen des Rückstandes mit je 200 ml Ethanol wird im Vakuumschrank bei 80 °C getrocknet.
Ausbeute: 67 g (66,4 %)
WS-Gehalt: 90 %

Die vereinigten Ethanolfiltrate werden soweit wie möglich eingeengt. Es werden 123,5 g Rückstand folgender Zusammensetzung erhalten:
9,6 g (0,04 Mol) Natriumacetoxibenzolsulfonat
91,5 g (0,58 Mol) Isononansäure
17,4 g (0,05 Mol) Natriumisononanoyloxibenzolsulfonat
2,0 g Natriumacetat
5,0 g Ethanol

2. Ansatz

64,0 g (0,4 Mol) Isononansäure
36,75 g (0,36 Mol) Acetanhydrid
58,85 g (0,3 Mol) Natriumphenolsulfonat
123,5 g Rückstand aus Ansatz 1

Es werden alle Komponenten außer Acetanhydrid zusammengegeben. Beim Erhitzen auf 130 °C destilliert restliches Ethanol ab. Acetanhydrid wird dann zugegeben. Die weitere Reaktionsführung und Aufarbeitung erfolgt wie in Ansatz 1.
Ausbeute: 84 g
WS-Gehalt: 92 %
Ges. Ausbeute nach 2 Ansätzen: 151 g Natriumisononanoyloxibenzolsulfonat (74,8 % bezogen auf 0,6 Mol Natriumphenolsulfonat)
Rückstand aus Ethanol: 154,86 g
bestehend aus:
12,5 g (0,05 Mol) Natriumacetoxybenzolsulfonat
26,5 g (0,08 Mol) Natriumisononanoyloxibenzolsulfonat
105,86 g (0,67 Mol) Nonansäure
2,0 g Natriumacetat
8,0 g Ethanol

3. Ansatz

64,00 g (0,4 Mol) Isononansäure
36,75 g (0,36 Mol) Acetanhydrid
58,85 g (0,8 Mol) Natriumphenolsulfonat
154,86 g Rückstand aus Ansatz 2
Ausbeute: 95 g
WS-Gehalt: 89 %
Ges. Ausbeute nach 3 Ansätzen: 246 g Natriumisononanoyloxibenzolsulfonat (81,1 % bezogen auf 0,9 Mol Natriumphenolsulfonat)
Rückstand aus Ethanol: 162,3 g
bestehend aus:
12,75 g (0,05 Mol) Natriumacetoxybenzolsulfonat
27,65 g (0,08 Mol) Natriumisononanoyloxibenzolsulfonat
116,9 g (0,74 Mol) Nonansäure
2,0 g Natriumacetat
3,0 g Ethanol

**Beispiel 4**

Natriumbenzoyloxibenzolsulfonat

Unter Stickstoff (siehe 1.) werden 58,85 g (0,3 Mol) Natriumphenolsulfonat, 146,5 g (1,2 Mol) Benzoesäure, 36,75 g (0,36 Mol) Acetanhydrid und 2 g Natriumacetat während 100 min auf 170 °C erhitzt und 2 h bei dieser Temperatur gehalten. Während 3 h wird auf 225 °C erhitzt. Dabei destilliert Essigsäure fast vollständig über. Es wird noch 10 min bei dieser Temperatur gehalten und während des Abkühlens der Rückstand mit 200 ml Methanol versetzt, 10 min gerührt und abfiltriert. Nach nochmaligem Waschen des Rückstandes mit 200 ml Methanol wird im Vakuumschrank bei 80 °C getrocknet.

Ausbeute: 85,6 g (95 %)
WS-Gehalt: 98 % (HPLC)

Die vereinigten Methanolfiltrate werden soweit wie möglich eingeengt. Es werden 116,4 g Rückstand erhalten bestehend aus
109,9 g (0,9 Mol) Benzoesäure
4,5 g (0,015 Mol) Natriumbenzoyloxibenzolsulfonat
2,0 g Natriumacetat

## Ansprüche

1. Verfahren zur Herstellung von Acyloxibenzolsulfonsäuren oder deren Salzen der Formel

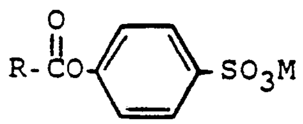

worin R $C_5$-$C_{21}$-Alkyl, $C_5$-$C_{21}$-Alkenyl, α-mono-Chlor-$C_5$-$C_{21}$-Alkyl, ω-$C_1$-$C_4$-Alkoxi-$C_5$-$C_{21}$-alkyl oder Phenyl, das durch 1 bis 3 Substituenten aus der Gruppe F, Cl, $SO_3M$, COOM, $C_1$-$C_{21}$-Alkyl und $C_2$-$C_{20}$-Alkenyl substituiert sein kann, und M Wasserstoff, Ammonium, ein Alkalimetallatom oder das Äquivalent eines Erdalkalimetallatoms bedeuten, durch Umsetzung einer Hydroxibenzolsulfonsäure oder deren Salzen der Formel

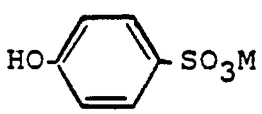

mit Acetanhydrid und einer Carbonsäuren der Formel
R-COOH
wobei R und M die oben genannten Bedeutungen haben, unter gleichzeitigem Abdestillieren der entstehenden Essigsäure und anschließender Isolierung der Acyloxibenzolsulfonsäure, oder deren Salzen, dadurch gekennzeichnet, daß man nach Beendigung der Reaktion die Acyloxibenzolsulfonsäure oder deren Salze durch Waschen des Reaktionsgemisches mit einem hydrophilen Lösemittel isoliert.

2. Verfahren nach Anspruch 1, dadurch gekennzeichnet, daß man Acyloxibenzolsulfonsäuren der angegebenen Formel herstellt, wobei R $C_5$-$C_{11}$-Alkyl, $C_5$-$C_{11}$-Alkenyl, α-mono-Chlor-$C_5$-$C_{11}$-Alkyl, ω-$C_1$-$C_4$-Alkoxi-$C_5$-$C_{11}$-alkyl oder Phenyl bedeutet.

3. Verfahren nach Anspruch 1, dadurch gekennzeichnet, daß man die Reaktion in Gegenwart katalytischer Mengen eines Alkali- oder Erdalkalisalzes einer Carbonsäure durchführt.

4. Verfahren nach Anspruch 1 oder 2, dadurch gekennzeichnet, daß man Hydroxibenzolsulfonat, Acetanhydrid und Carbonsäure im molaren Verhältnis 1 : 1-2 : 1-5 umsetzt.

5. Verfahren nach einem der Ansprüche 1 bis 3, dadurch gekennzeichnet, daß man die Reaktion bei 120 bis 250° C durchführt.

6. Verfahren nach einem der Ansprüche 1 bis 4, dadurch gekennzeichnet, daß man als hydrophiles Lösemittel einen $C_1$-$C_3$-Alkohol nimmt.

Europäisches Patentamt

# EUROPÄISCHER RECHERCHENBERICHT

Nummer der Anmeldung

EP 89113007.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | EP - A1 - 0 105 672 (THE PROCTER & GAMBLE) * Ansprüche 1,12 * -- | 1 | C 07 C 143/38<br>C 07 C 139/00 |
| D,X | EP - A1 - 0 098 129 (THE PROCTER & GAMBLE) * Beispiel 1 * -- | 1,2,6 | |
| A | EP - A1 - 0 270 304 (INTEROX CHEMICALS) * Anspruch 1 * -- | 1 | |
| D,A | EP - A1 - 0 207 445 (HOECHST) * Ansprüche 1,3,5 * -- | 1 | |
| D,A | EP - A1 - 0 153 223 (RHONE-POULENC CHIMIE DE BASE) * Ansprüche 1,6 * -- | 1,3 | |
| D,A | EP - A2 - 0 105 673 (PROCTER & GAMBLE) * Anspruch 1 * -- | 1 | RECHERCHIERTE SACHGEBIETE (Int. Cl.4) |
| D,A | EP - A2 - 0 125 641 (ETHYL CORPORATION) * Anspruch 1 * -- | 1 | C 07 C 143/00<br>C 07 C 139/00<br>C 11 D |
| A | US - A - 4 544 503 (C. BERNARD BERRY) * Zusammenfassung * ---- | 1 | |

Der vorliegende Recherchenbericht wurde für alle Patentansprüche erstellt.

| Recherchenort | Abschlußdatum der Recherche | Prüfer |
|---|---|---|
| WIEN | 13-10-1989 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument